# EUROPEAN PATENT APPLICATION

(11) **EP 1 002 512 A2**
(43) Date of publication of application: **24.05.2000**
(21) Application number: 99103492.7
(22) Date of filing: 23.02.1999
(51) Int. Cl.: A61J 1/10

(54) **Flexible container for the containment and delivery of fluids**

(30) Priority: 19.11.1998 US 196084
(71) Applicant: Bracco International B.V., 1077 ZX Amsterdam (NL)
(72) Inventor: Cornille, John J., Skillman, NJ 08558 (US); Niedospial, John J. Jr., Burlington, NJ 08016 (US)
(74) Representative: Gervasi, Gemma, Dr.

(57) **Abstract**

Unitary, flexible container for the containment and delivery of medical fluids, having first (36, 66, 96, 126, 156) and second (38, 68, 98, 128, 158) polymeric sheets superimposed and sealed together at their periphery to form a pouch (10, 40, 70, 100, 130), wherein the first and second sheets are non-coplanar to each other and form a concavo-convex shape reservoir.

## Description

### Field of the Invention

The present invention relates to flexible containers for receiving, containing and delivering fluids such as blood products, parenteral solutions, drug formulations and diagnostic contrast media.

More particularly the invention relates to flexible containers essentially of thermoplastic materials that maintain their intended shape after being filled or partially filled with a fluid.

### Background of the invention

Prior to the discovery and development of polymeric materials, parenteral liquids have been supplied to hospitals exclusively in glass bottles. The disadvantages of glass bottles, such as cost, shipping, storage and disposal, prompted the prior art to provide flexible, sterilizable containers in the forms of bags and bottles for the containment and delivery of parenteral solutions, such as diagnostic contrast media, nutritional and drug formulations. Such containers typically comprise: a flexible plastic sheet formed into a pouch, bag or bottle shape filled with a solution inside therein in a sterile environment; and one or more ports to fill and/or access the solution. Flexible tubing is also provided one end of which is connectable to a port on the container, and the other end connectable to a syringe or catheter pre-inserted into the site of delivery on the patient. Control means are also usually included with the tubing, such as valves and clamps for initiating, controlling and terminating the flow of the liquid to the delivery site. The container, tubing and control means are sterile packaged ready for use.

The desiderata of flexible containers for receiving, containing and delivering medical products include inertness of the containers to the medical products, air and fluid impermeability, stability on storage, preventative ability to UV rays from the outside environment, lack of "hold-up" of the fluid on the walls of the containers so that the intended amount of fluid is delivered to the patient, maintenance of the shape of the container after the same have been filled or partially filled so that the volume of fluid administered or received at any particular time can be visually observed by the practitioner.

The present invention is particularly directed to complete delivery of medical fluid to a site of administration without "hold-up" of drops on the walls of the containers when filled or partially filled with a medical fluid.

One of the two requirements particularly addressed by the present invention is that the flexible containers for delivering parenteral solutions to patients deliver, by their construction and design, the total contents in a uniform, steady manner without retaining fluid drops on their walls. By meeting such requirement the medical practitioner can determine the amount of parenteral solution delivered from the container to the patient. The prior art has addressed this requirement, as shown for example in U.S. Patent No. 4,892,537, which discloses a bag having substantially parallel major sides or edges and converging minor sides which meet at a point forming an obtuse angle of at least 110(. The converging edges are designed to guide the filled bag contents in a substantially unobstructed manner in a funnel-like fashion to an exit port.

U.S. Patent No. 4,088,166 also addresses the problem of incomplete and non-uniform collapse of parenteral solution containers. The incomplete collapse is attributed to the stiffness of the thin-walled polypropylene container which tends to resist collapse to such a degree that the moderate suction pressure exerted on the container by weight of the parenteral solution is insufficient to cause its complete collapse. The non-uniform collapse, on the other hand, is attributed to the observed facts that on some occasions, the bags collapse along the long axis of their cross section, while on other occasions they tend to collapse along both the short axis of the cross-section, as well as the long axis. As a result, the medical practitioners cannot determine exactly how much parenteral solution has been delivered out from the container. In order to solve the problem of incomplete and non-uniform delivery , the patentee incorporates gusset portions in the body portion of the container adjacent the shoulder portion. The gusset portions include lines of flexing weakness to facilitate the collapse of the container adjacent the shoulder portion as the contents thereof are withdrawn. The gussets said to facilitate both the lateral and longitudinal collapse of the container as it is emptied.

Medical practitioners have also observed fluid "hold-up", i.e. when drops of parenteral solutions tend to remain on the internal walls of the flexible container as the solution is being delivered to the site of administration. The moderate suction pressure exerted on the walls of the container is insufficient to overcome the force existing between the drops of liquid and the walls of the container. Often, as the container is being drained, the emptied portion of the parallel walls adhere to each other further trapping drops of the liquid. As a consequence, the prescribed amount of parenteral solution is not delivered to the patient. Such delivery, especially in traumatic circumstances where a precise amount of drug must be delivered into the patient, can make the difference between life and death of the patient.

U.S. Patent No. 5,738,671 addresses the need for complete delivery of medical fluids from flexible plastic containers, such as bags and bottles, without "hold-up" of fluid drops on the internal walls of the containers by providing flexible plastic containers the interior walls of which are mechanically or chemically embossed to maintain the polymeric sheets forming the interior reservoir spaced apart from each other thereby allowing relative movements of the polymeric sheets and preventing sticking therebetween. The embossments disclosed can be in the form of checkerboard, micro circles or dots, vertical channels and S-shaped channels.

The other particular requirement addresses by the present invention is the maintenance of the shape or configuration of the flexible containers when filled or partially filled with a medical fluid. The prior art provides various geometric shapes and reinforcements in plastic containers to satisfy this requirement. For example: U.S. Patent No. 5,174,458 discloses a collapsible container having a plurality of transversely extending grooves in the front and rear panels to minimize outward bulging when the container is filled; U.S. Patent No. 3,926,341 pertains to bottles of semi-rigid plastic material wherein the bottles are of concave polygon shape the concavity of which increases when, under slight internal depression, the walls tend to join together.

We have now discovered that these two requirements, in addition to other desirable characteristics, can be met in a flexible container that maintains its predetermined configuration without the use of reinforcing means when filled, or partially filled, with a fluid wherein the walls of the reservoir formed by two non-coplanar sheets.

### Summary of the invention

The present invention is directed to a flexible, unitary plastic container, such as a bag, pouch or bottle, for the containment and delivery of fluids, such as parenteral solutions including diagnostic contrast media, drug formulations and nutrients to a patient in need of such fluids. The flexible, unitary plastic container also finds utility in receiving and maintaining blood and blood components which are frozen after collection and are maintained at temperatures of -30° to -80°C.

The flexible unitary plastic container may be of any configuration such as, for example, square, round, oval, rectangular, hexagonal or octagonal. In all the configurations the container is formed of first and second non-coplanar polymeric sheets superimposed and sealed together at their periphery to form an interior reservoir, forming a concavo-convex shape, for receiving, maintaining or delivering a fluid. The container has one or more access ports for allowing filling of the container with a fluid and access thereto for its delivery. Typically a flexible tubing one end of which is integral with the access port is provided in the container for attachment of a delivery means.

Further aspects of the invention provides for containers, according to the invention, having a different configuration.

In reference to a generally rectangular and square configuration, the container comprises:
a) first and second flexible plastic sheets having a generally rectangular or square configuration superimposed and sealed together at their periphery to form a pouch defining an interior reservoir, said pouch having a top and bottom portion, wherein said first or second sheets are non-coplanar to each other forming a concavo-convex shape; said bottom portion terminates in a first angle and a second angle of from about 5° to 45°, preferably of from about 10° to about 30°, and more preferably from 10° to 20°, from the centre of said bottom portion and relative to a horizontal plane crossing the centre of said bottom portion to direct and facilitate the flow of content contained in the pouch towards an access port;
b) at least one access member integral with said pouch, preferably said access member is located at the centre of the bottom portion of said pouch allowing filling of the pouch wit a fluid and access thereto for its delivery, said access member comprising:
   at least one access port located below the bottom portion of said pouch where said first angle and said second angle meet.

In reference to a generally hexagonal or octagonal configuration, the container comprises:
a) first and second plastic sheet having a generally hexagonal or octagonal configuration superimposed and sealed together at their periphery to form a pouch defining an interior reservoir forming a concavo-convex shape, said pouch having a top portion at an angle of the hexagonal or octagonal configuration and a bottom portion at another angle of the hexagonal or octagonal configuration, said top and bottom angles being on opposite sides of each other at the longest distance within the confines of the hexagonal or octagonal configuration, wherein said first and second sheets are non-coplanar to each other; and
b) an access member integral with said pouch, said access member preferably is located at the centre of the bottom portion of said pouch allowing filling of the pouch with a fluid and access thereto for its delivery, said access member comprising:
   an access port located in said access member.

In reference to a generally parabolic configuration, the flexible unitary container comprises:
a) first and second collapsibly thin polymeric sheets having a generally parabolic side configuration superimposed and sealed together at their periphery to form:
   1) an interior reservoir of a concavo-convex's shape;
   2) a non-collapsible top portion;
   3) a non-collapsible bottom portion, and
   4) parabolic side portions; and
b) an access member;
wherein said first and second sheets are non-coplanar to each other.

According to a preferred aspect, said top portion comprises:
a centre area where the polymeric sheets sealed together form a hole for suspending the bag during delivery of its content;
two symmetrically positioned rectangular areas extending outwardly from the centre of the bag and sealed at their periphery to render said top portion less flexible than the polymeric sheets which form the interior reservoir; and
said bottom portion comprises:
two symmetrically positioned rectangular areas extending outwardly from the centre of the bag and sealed at their periphery to render said bottom portion less flexible than the polymeric sheets which form the interior reservoir, said interior reservoir at its bottom portion terminates in a first angle and a second angle of from about 5° to about 45° each from the centre thereof and relative to a horizontal plane crossing the centre of said bottom portion;
said parabolic side portions are designed to flex inwardly when the medical bag is filled with a parenteral solution;
said access member located in the centre of the bottom portion where said first angle and said second angle meet.

In reference to a generally oval or elliptical configuration, the container comprises:
a) first and second plastic sheet having a generally oval or elliptical configuration superimposed and sealed together at their periphery to form a pouch defining an interior reservoir forming a concavo-convex shape, said pouch having a top portion and a bottom portion, wherein said first and second sheets are non-coplanar to each other; and
b) an access member integral with said pouch, preferably said access member is located at the centre of the bottom portion of said pouch allowing filling of the pouch with a fluid and access thereto for its delivery, said access member comprising: an access port located in said access port.

In reference to a generally spherical configuration, the flexible unitary container of the present invention comprises:
a) a first and second flexible plastic sheet having a generally spherical configuration superimposed and sealed together at their periphery to form a pouch defining an interior reservoir, said pouch having a top and bottom portion, wherein said first and second sheets are non-coplanar to each other forming a concavo-convex shape; and
b) an access member integral with said pouch, preferably said access member is located at the centre of the bottom portion of said pouch allowing filling of pouch with a fluid and access thereto for its delivery, said access member comprising:
   an access port located in said access member.

Preferably, the top portion at the periphery of the pouch according to any configuration of the invention comprises at least one hole for suspending the pouch when it is in use for delivering the content thereof to the delivery site; and the bottom portion at the periphery of the pouch comprises one or more holes to facilitate suspending the pouch during the filling process.

The flexible unitary container is three dimensional prior to the introduction of fluid thereinto: each of the two superimposed sheets is pre-formed into a concavo-convex shape, the concave surface being towards the content of the reservoir while the convex surface faces towards the outside.

In preparing the flexible unitary container of the present invention feeding material supplied in rolls of single or multi-layered polymeric sheet film is introduced into a cavity of desired three dimensional shape, such as rectangular, square, oval, spherical, hexagonal and octagonal. The cavity is prefabricated from a suitable material such as stainless steel. Small holes are present throughout the surface of the cavity which are connected to a vacuum source. The polymeric sheet is fed into the cavity on the top surface thereof and heated to a temperature to soften the sheet to a flowable consistency. By applying the vacuum the sheet conforms to the shape of the cavity. The sheet, having the three dimensional shape is allowed to cool to a temperature at which it no longer flows, and is removed from the cavity.

The flexible unitary container is formed by superimposing two pre-formed sheets on top of one another so that their concave surface face each other in a minor image fashion, then applying heat and pressure to their peripheral areas to fuse the sheets together.

In addition to vacuum forming other methods, such as blow moulding, may be used to manufacture the flexible unitary container of the present invention.

The sheets forming the container of the present invention may be multilayers. The sheets may include UV rays impermeable films for preventing degradation of light sensitive fluids contained in the container. The walls of the container may be made of a sandwich of polymeric films enclosing a UV rays barrier opaque layer such as a metal foil to prevent degradation of light sensitive fluid contents. The UV barrier opaque layer should have a "window" through which the content of the container could be observed.

Additional features and advantages of the present invention will be apparent from the detailed description and the drawings.

### Brief Descritpion of the Drawings

FIG. 1 is a front elevational view of a flexible unitary container of the present invention in the form of a generally rectangular pouch configuration, the back elevational view being identical with the front elevational view thereof;
FIG. 2 is a right-side elevational view of the flexible unitary container of FIG. 1, the left-side elevational view being identical with the right-side elevational view thereof;
FIG. 3 is a front elevational view of another embodiment of the flexible unitary container of the present invention in the form of a generally hexagonal pouch configuration, the back elevational view being identical with the front elevational view thereof;
FIG. 4 is a right-side elevational view of the flexible unitary container of FIG. 3, the left-side elevational view being identical with the fight-side elevational view thereof;
FIG. 5 is a front elevational view of another embodiment of the flexible unitary container of the present invention in the form of a generally parabolic pouch configuration, the back elevational view being identical with the front elevational view thereof;
FIG. 6 is a right-side elevational view of the flexible unitary container of FIG. 5, the left-side elevational view being identical with the right-side elevational view thereof
FIG. 7 is a front elevational view of another embodiment of the flexible unitary container of the present invention in the form of a generally oval pouch configuration, the back elevational view being identical with the front elevational view thereof;
FIG. 8 is a right-side elevational view of the flexible unitary container of FIG. 7, the left-side elevational view being identical with the right-side elevational view thereof;
FIG. 9 is a front elevational view of another embodiment of the flexible unitary container of the present invention in the form of a generally spherical pouch configuration, the back elevational view being identical with the front elevational view thereof; and
FIG. 10 is a right-side elevational view of the flexible unitary container of FIG. 9, the left-side elevational view being identical with the right-side elevational view thereof.

### Detailed Description of the Invention

The present invention provides a flexible plastic container, in the form of a bag, pouch or bottle, for the containment and delivery of fluids, such as diagnostic contrast media, nutrients and drug formulations. The configuration of the flexible plastic container may be: polygon, such as rectangular, square, hexagonal and octagonal; spherical polygon; spheroidal; and ellipsoidal. Preferred configurations are rectangular, oval, hexagonal, parabolic and spherical.

In all the configurations of the present invention shown in the drawings and/or referred to in the specification the flexible plastic container comprises two preformed sheets which are not coplanar and not parallel to each other. The sheets are superimposed on each other and sealed together at their periphery to form a reservoir for the containment of fluid. Contrary to prior art containers wherein flat, coplanar sheets are welded together at their periphery forming an essentially two dimensional reservoir prior to the introduction of a fluid thereinto, the flexible plastic container of the present invention is three dimensional prior to the introduction of fluid thereinto: each of the superimposed sheets is concavo-convex, the concave surface being towards the content of the reservoir while the convex surface faces towards the outside. Although being in a collapsed state, the container has, in addition to its length and width, a depth separating the two superimposed sheets. Upon filling the reservoir the container assures the pre-formed shapes of the two non-coplanar concavo-convex sheets.

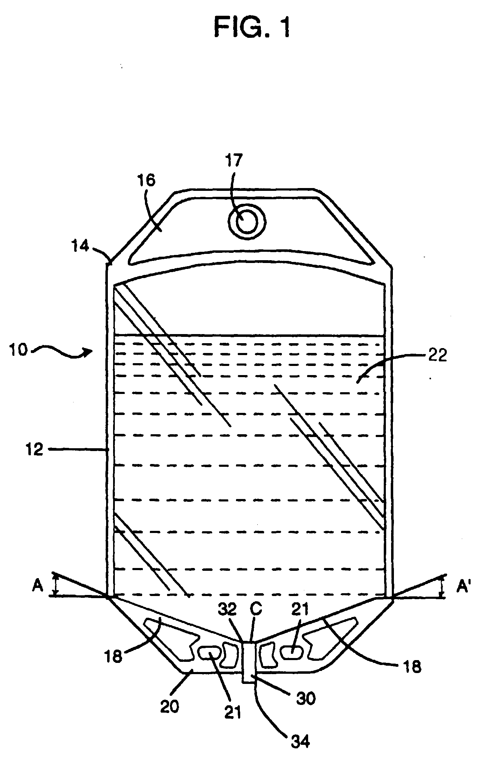

Referring to the drawings, FIG. 1 shows in a front elevational view a generally rectangular, flexible, transparent plastic pouch generally designated at 10 partially filled with fluid 22. The pouch comprises at least one hole 17 for suspending when it is used for delivering its fluid content. In its filled or partially filled state, when suspended, the pouch assumes the shape of a small cushion; while in an unfilled and suspended state the three dimensional configuration of the pouch is less pronounced.

The flexible pouch 10 comprises pre-formed superimposed sheets joined together by heat sealing means along marginal areas 12, 14, 16, 18 and 20. Preferably the bottom portion of pouch 10 terminates in a first angle A and a second angle A' from the centre C and relative to a horizontal plane crossing the centre C of said bottom portion to direct and facilitate the flow of content 22 contained in the pouch towards an access port. Angles A and A' are of from about 5° to about 45°, preferable from 10° to 30°, and most preferably from 10° to 20°.

An access port 30 located at centre C of the bottom portion of the pouch 10 is sealed between the first sheet and the second sheet of the pouch comprising a top, liquid-contacting portion 32 and a bottom portion 34 to which access means, such as an intravenous line can be permanently or fixedly attached by heat sealing or by other means. Access port 30 serves for both the filling and for the delivery of parenteral fluids, such as contrast media and drug formulations. Marginal area 16 comprises at least one hole 17 for suspending the pouch when used for delivering the content thereof to a delivery site. Marginal area 20 comprises at least one and preferably a plurality of holes 21 to facilitate suspending the pouch during the filling process.

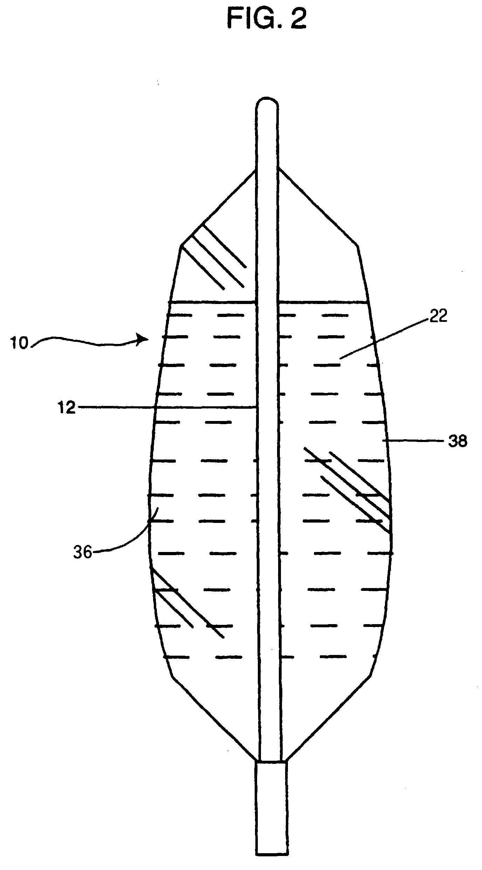

FIG. 2 shows a right-side elevational view of the flexible plastic pouch shown in FIG. 1, the left-side elevational view being identical with the right-side elevational view thereof. As shown by the side elevational view the sheets 36 and 38 forming the pouch 10 are spaced from each other while the pouch is filled or partially filled with a fluid. The spacing of the sheets is less pronounced prior to the pouch being filled with a fluid.

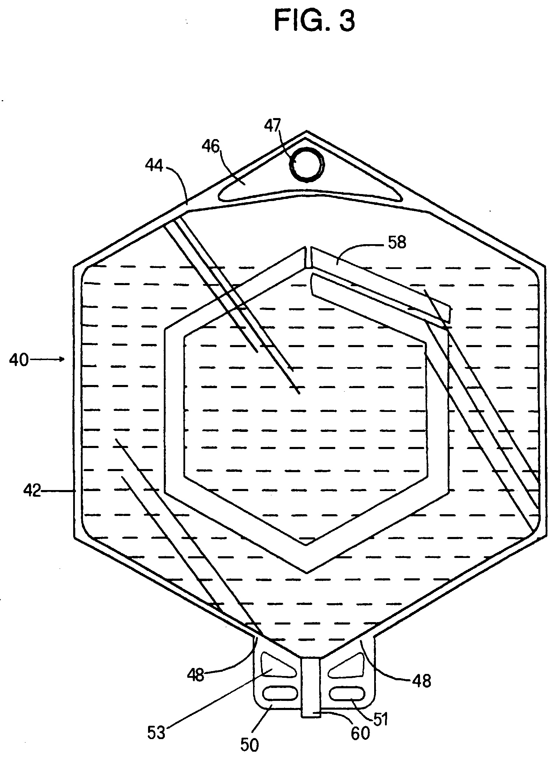

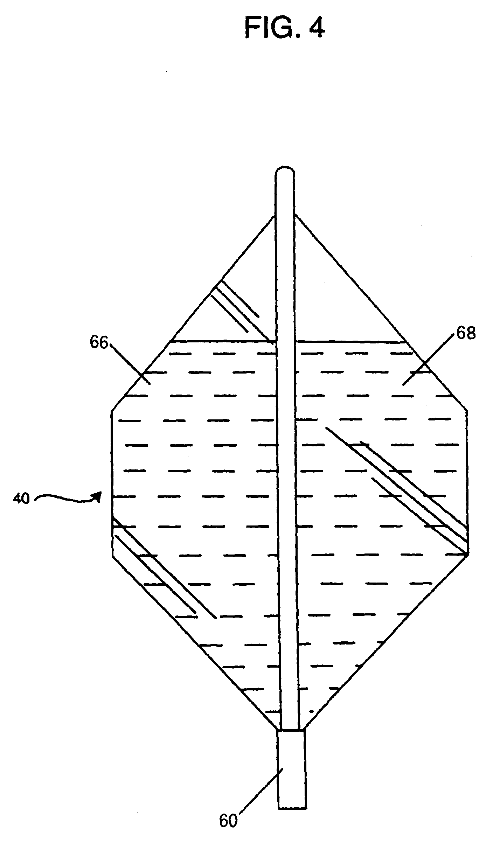

FIGS. 3 and 4 show a second embodiment of the flexible unitary container in the form of a generally hexagonal pouch configuration: FIG. 3 showing a front elevational view and FIG. 4 showing a right-side elevational view thereof. The back elevational view is substantially identical with the front elevational view, while the left-side elevational view is identical with the right-side elevational view.

The hexagonal pouch 40 comprises preformed superimposed sheets joined together by heat sealing means along marginal areas 42, 44, 46, 48 and 50. The bottom portion by the configuration of the hexagon directs the flow of fluid content 22 to access port 60 which is sealed between first sheet 66 and second sheet 68. Access port comprises top liquid contacting portion 62 and bottom portion 64. Access port 60 serves for both the filling and the delivery of fluids, such as contrast media and drug formulations. Marginal area 46 comprises at least one hole 47 for suspending the pouch when used for delivering the content thereof to a delivery site. Marginal area 50 comprises at least one and preferably a plurality of holes 51, 53 to facilitate suspending the pouch during the filling process.

A hexagonal logo 58 is optionally printed on the front or back or both sides of the pouch.

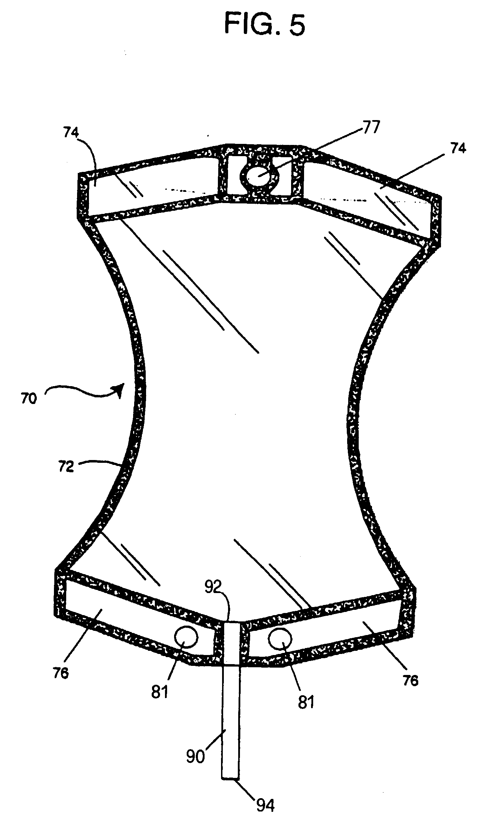

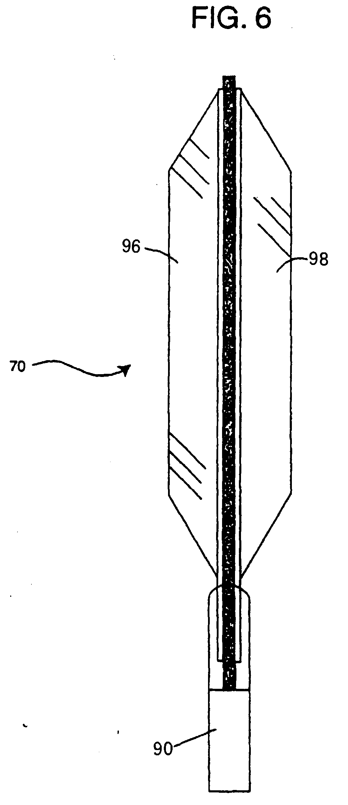

FIG. 5 shows in a front elevational view a third embodiment of the flexible unitary container of the present invention in the form of a generally parabolic pouch configuration, the back elevational view being identical with the front elevational view thereof; and FIG. 6 show a right-side elevational view of the pouch of FIG. 6, the left-side elevational view being identical with the right-side elevational view thereof.

The pouch 70 comprises preformed superimposed sheets 96 and 98 joined together by heat sealing means along marginal areas 72, 74 and 76. Fluid content flows toward access port 90, which is sealed between the superimposed sheets. Access port 90 comprises top liquid contacting portion 92 and bottom portion 94. The access port serves both for filling the pouch and delivering of fluids to a site. Marginal area 74 comprises at least one hole 77 for suspending the pouch when used for delivering the content thereof to a delivery site. Marginal area 76 comprises at least one and preferably a plurality of holes 81 to facilitate suspending the pouch during the filling process.

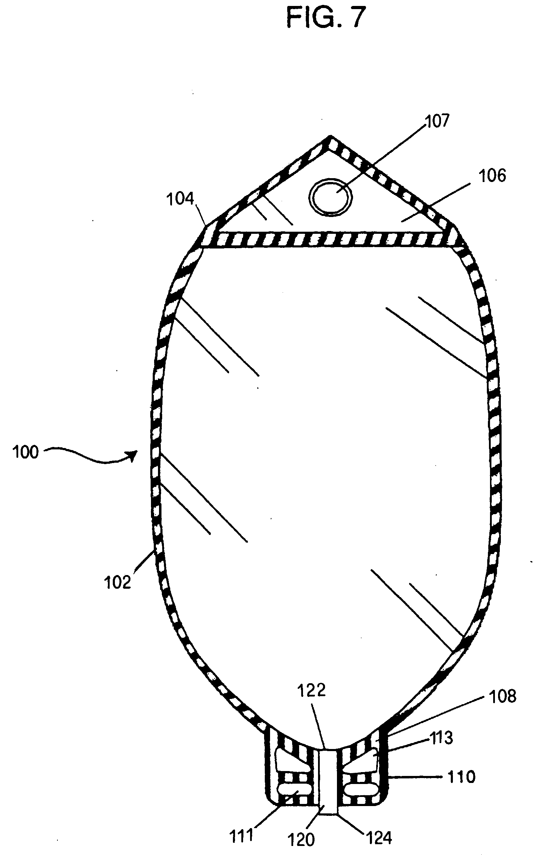

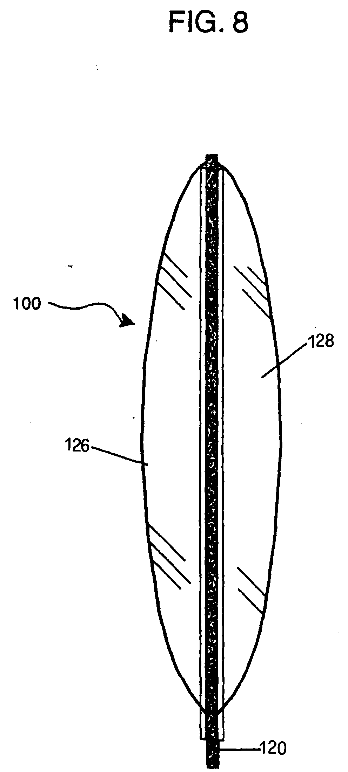

FIG. 7 shows in a front elevational view a fourth embodiment of the flexible unitary container of the present invention in the form of a generally oval or elliptical pouch configuration, the back elevational view being identical to the front elevational view thereof; and FIG. 8 shows a right-side elevational view of the pouch shown in FIG. 7, the left-side elevational view being identical with the right-side elevational view thereof.

The pouch 100 comprises preformed superimposed sheets 126 and 128 joined together by heat sealing means along marginal areas 102, 104, 106, 108 and 110. The bottom portion of the pouch is provided with an access port 120 which is sealed between the pre-formed superimposed sheets 126 and 128. Access port 120 comprises a top liquid contacting portion 122 and bottom portion 124. The access port serves for the filling and the delivery of fluids into and out of the pouch. Marginal area 110 comprises at least one and preferably a plurality of holes 111 and 113 to facilitate suspending the pouch during the filling process. Marginal area 106 comprises at least one hole 107 for suspending the pouch when it is used for delivering the content thereof to a delivery sight.

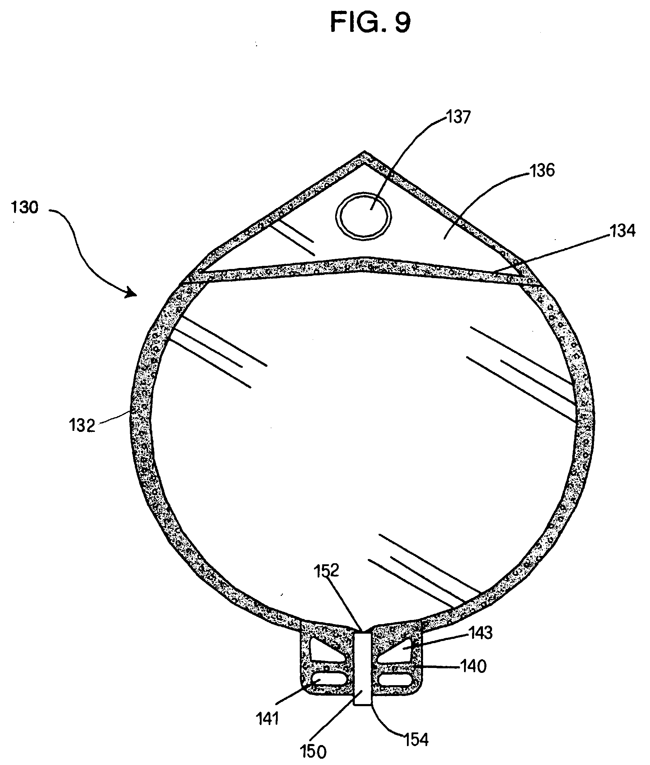

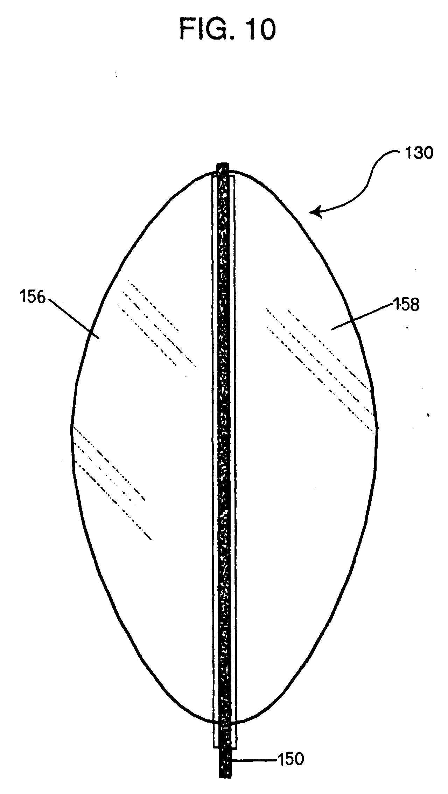

FIG. 9 shows in a front elevational view a fifth embodiment of the flexible unitary container of the present invention in the form of a generally spherical pouch configuration, the back elevational view being identical to the front elevational view thereof; and FIG. 10 shows a right-side elevational view of the pouch shown in FIG. 9, the left-side elevational view being identical with the right-side elevational view thereof.

The pouch 130 comprises preformed superimposed sheets 156 and 158 joined together by heat sealing means along marginal areas 132, 134, 136, 138 and 140. The bottom portion of the pouch is provided with an access port 150 which is sealed between the pre-formed superimposed sheets 156 and 158. Access port 150 comprises a top liquid contacting portion 152 and bottom portion 154. The access port serves for both the filling and delivery of fluids into and out of the pouch. Marginal area 140 comprises at least one and preferably a plurality of holes 141 and 143 to facilitate suspending the pouch during the filling process. Marginal area 136 comprises at least one hole 137 for suspending the pouch when it is used for delivering the content thereof to a delivery site.

### Materials Of Construction

The flexible container of the present invention is made of known polymeric materials having properties which make them suitable for sterile delivery of parenteral liquids. The sheets for forming the walls of the container are preferably multilayer sheets and characterized by heat resistance, gloss, strength, flexibility, and chemical inertness. Preferably the sheets are transparent or at least translucent enabling visual inspection of the contents at all time during the delivery of content from the container to the patient. The container must be sterilizable, preferably by heat, along with its content. At least one layer of the sheet must be impervious to atmospheric gases and to steam. Preferably, the internal surface of the pouch in contact with the parenteral solution should be impervious to gases and steam. The interior layer in contact with the parenteral solution must not contain any toxic agents or even plasticizers which could leach out and contaminate the solution. The sheet may be made, for example, from polyvinylidene chloride sandwiched between two polyethylene or polyvinylidene layers. The polyvinylidene chloride constitutes the impervious barrier. Further layers may be added to the face or back of the sheet, if desired, such as a polyolefin, preferably, polyethylene. Polyvinyl chloride is also suitable for the construction of the sheet and is well-accepted by the prior art for use in containers for medical fluid collection and delivery. Typical properties of polyvinyl chloride films include: a thickness of about 380 micron; a tensile strength of about 240 kg/cm2; a moisture vapor transmission rate of about 14-20 (g/m2/day at 38°C, 100%RH); and an oxygen barrier of 650 (cc/m2/day at 23°C, 0% RH, bar. CRYOVAC® sterilizable medical films (W.R. Grace and Co.) are especially suitable to construct the sheets used in the present invention. The films comprise a polyethylene layer sandwiched between polyester outer layers sealed together by a modified propylene copolymer. Typical properties of the film include: a thickness of about 190 micron; a tensile strength of about 250kg/cm2, a moisture vapor transmission rate of 5 (g/m2/day at 38°C, 100%RH); and an oxygen barrier of about 1500 (cc/m2/day at 23°C, 0%RH, bar).

Other preferred polymeric films or sheets for constructing the flexible container of the present invention include: copolyester ether monolayer films, such as polycyclohexanedimethylcyclohexane dicarboxylate elastomer made by Eastman Chem. Co.; and ethyl vinyl acetate made by Stedim, Inc. It is important that the fluid contacting layer of the multilayer sheet contain no plasticizer which may contaminate the fluid content of the container. Preferably, no plasticizer should be used at all on any of the multilayers to form a flexible container of the present invention.

The flexible container of the present invention may be made of clear flexible film having UV absorbing (scavenging) or oxygen absorbing (scavenging) properties so that the content of the container is not affected by these environmental conditions. These polymers include in the form of film alloys, blends, extrusions, laminations, surface modified and impregnated films or combinations thereof the following polymeric materials which are capable to withstand autoclave or high temperature sterilization and which contain UV absorbing or oxygen scavenging agent or blocking agents/ UV stabilizers, into which such agents are incorporated by processes known to those skilled in the art:
Copolyester elastomers, ethylene methacrylate, ethylene vinyl acetate, ethylene vinyl alcohol, low density polyethylene, nylon/polypropylene, polyester, polyolefin, polypropylene, polyethylene, and polyvinylchloride.
Blocking agents/UV stabilizers which may be included in the films include: N-(2-Aminoethyl)-3-aminopropylmethyldimethoxy silane; 3-Aminopropylmethyldiethoxy silane: Amyitrichloroilane;
Bis (hydroxyethyl) aminopropyltriethoxy silane; Bis-(N-methylbenzanide) ethoxymethyl silane; Bis(trimethylsilyl)acetamide;
3-Chloroproplytriethoxysilane;
Di-t-butoxdiacetoxysilane;
Ethyltriacetoxysilane;
(3-Glycidoxypropyl)-methyldiethoxy silane;
Isobutyltrimethoxysilane; Isocyanatopropyltriethoxysilane;
3-Mercaptopropylmethyldimethoxysilane; Mercaptopropyltrimethoxysilane; N-methylaminopropyltimethoxysilane; Methyltriacetoxysilane; Methyltriethoxysilane; Methyltrimethoxysilane;
Octyltriethoxysilane;
2-Phenylethyltrichlorosilane; Phenyltriethoxysilane; n-Propyltrimethoxysilane 3-(N-Styroylmethyl-2-aminoethylamino) propyltrimethoxy silane hydrochloride,
2-(3'-t-butyl-2'-hydroxy-5'-methylphenyl)-5-chlorobenzotriazole,
Bis (1,2,2,6,6,-pentamthyl-4-piperidinyl)3,5-di-butyl-4-hydroxybenzyl)butyl propanedioate and ethyl 2-cyano-3,3-diphenylacrylate.

Access port and tubing used in the present invention may be made of polyvinyl chloride which are sold commercially for use in medical devices. Other port and tubing materials may also be used, such as CRYOVAC® Port Tubing (W.R. Grace & Co.) which comprise three concentric layers of polymeric materials: a polyolefin layer is sandwiched between an outer layer of modified propylene copolymer and an inner layer of ethylene vinyl acetate or polyvinyl chloride.

### The Process of Making the Flexible Container of the Present Invention

The manufacturing process of making the flexible container comprises the steps of:
forming thermoplastic sheets into desired three dimensional shapes; and
heat sealing the marginal areas to form the container.

The thermoplastic sheets of single or multi-layered films are optionally pre-heated to a pliable state and placed over cavities which were pre-made to desired shapes, such as square, rectangular, spherical and the like. The cavities are made of steel or other suitable material. The pre-heating may be accomplished by using various techniques known in the art, such as blowing hot air over the sheets or drawing the sheets across a heated platen or bar. The cavity used may be a male or female cavity equipped with small holes through which vacuum can be applied. In either case the cavity has one half of the desired shape of the finished container. A series of these halves are then superimposed and sealed together at their marginal areas to form the container.

The pre-heated sheet is fed over the top surface of the cavity and heated to a temperature at which it softens and is able to flow into the cavity. As the vacuum is applied, air between the wall of the cavity and the soft, flowable sheet is exhausted causing the sheet to be drawn into the cavity and conform to the cavity's configuration. The so-formed three dimensional sheet is then allowed to cool to a temperature at which it no longer flows and is removed from the cavity. Alternatively the pre-formed sheets may be produced by using both a male and female configuration cavity, the female cavity being provided with a plurality of small holes connected to a vacuum source. The thermoplastic sheet is heated and simultaneously pulled into the female cavity and pushed by the male cavity having a smaller dimension. Vacuum is then applied to the female cavity to exhaust any air that might be trapped between the sheet and the surface of the female cavity. Still more alternatively, the male cavity may be provided with a plurality of holes through which air can be supplied forcing the thermoplastic sheet into the female cavity to assume its configuration.

In a still further alternative process thermoplastic material is placed in a mould having a desired configuration, heated past its softening point, and applying pressurised gas to expand the thermoplastic material to assume the configuration of the mould. The thickness of the finished product can be controlled by the amount of thermoplastic material placed in the mould.

Subsequent to forming a series of three dimensional halves, two symmetrical halves are superimposed on each other and heat and pressure are applied to their marginal areas to fuse the two halves together to form a hermetic seal between them. One ore more exit ports are introduced between the two halves of the container during the joining process for providing access to fill or empty the container. Desirably, holes are punched into the top and bottom perimeter areas of the container to facilitate suspension of the container for filling or delivering its content.

### List of Reference Numbers Used

### First embodiment

| | |
|---|---|
| Pouch | 10 |
| Fluid in pouch | 22 |
| Hole on top portion of pouch for suspending same when delivering of fluid | 17 |
| Hole for suspending the pouch during the filling process | 21 |
| First and Second superimposed sheets | 36 and 38 |
| Marginal areas of sheets joined together | 12,14,16,18 and 20 |
| Access port | 30 |
| Fluid contacting portion of access port | 32 |
| Bottom portion of access port | 34 |

### Second embodiment

| | |
|---|---|
| Pouch | 40 |
| Hole on top portion of pouch for suspending same when delivering fluid | 47 |
| Hole for suspending the pouch during the filling process | 51, 53 |
| First and second superimposed sheets | 66 and 68 |
| Marginal areas of sheets joined together | 42,44,46,48 and 50 |
| Access port | 60 |
| Fluid contacting portion of access port | 62 |
| Bottom portion of access port | 64 |
| Hexagonal logo printed on pouch | 58 |

### Third embodiment

| | |
|---|---|
| Pouch | 70 |
| Hole for suspending the pouch when delivering fluid | 77 |
| Holes for suspending the pouch during the filling process | 81 |
| First and second superimposed sheets | 96 and 98 |
| Marginal areas of sheets joined together | 72,74 and 76 |
| Access port | 90 |
| Fluid contacting portion of access port | 92 |
| Bottom portion of access port | 94 |

### Fourth embodiment

| | |
|---|---|
| Pouch | 100 |
| Hole for suspending the pouch when delivering fluid | 107 |
| Holes for suspending the pouch during the filling process | 111, 113 |
| First and second superimposed sheets | 126 and 128 |
| Marginal areas of sheets joined together | 102,104,106,108,110 |
| Access port | 120 |
| Fluid contacting portion of access port | 122 |
| Bottom portion of access port | 124 |

### Fifth embodiment

| | |
|---|---|
| Pouch | 130 |
| Hole for suspending the pouch when delivering fluid | 137 |
| Holes for suspending the pouch during the filling process | 141, 143 |
| First and second superimposed sheets | 156 and 158 |
| Marginal areas of sheets joined together | 132,134,136,138,140 |
| Access port | 150 |
| Fluid contacting portion of access port | 152 |
| Bottom portion of access port | 154 |

Various modifications of the several embodiments disclosed will become apparent to those skilled in the art. The invention is intended to include such modifications to be limited only by the scope of the claims

## Claims

1. A unitary, flexible container made of a polymeric material for the containment and delivery of a medical fluid comprising:
a) first (36, 66, 96, 126, 156) and second (38, 68, 98, 128, 158) flexible polymeric sheets superimposed and sealed together at their periphery to form a pouch (10, 40, 70, 100, 130) defining an interior reservoir, said pouch having a top and bottom portion, wherein said first and second sheets are non-coplanar to each other forming a concavo-convex shape;
b) at least one access member integral with said pouch.

2. A container according to claim 1, wherein said first (36) and second (38) flexible polymeric sheets have a generally rectangular or square configuration and form said pouch (10).

3. A container according to claim 2, wherein
a) said first (36) and second (38) flexible polymeric sheets are superimposed and sealed together at their periphery (12, 14, 16, 18, 20) to form said pouch (10) defining an interior reservoir, said pouch having a top and bottom portion, said bottom portion terminates in a first angle and a second angle of from about 5° to 45° from the centre of said bottom portion and relative to a horizontal plane crossing the centre of said bottom portion to direct and facilitate the flow of content (22) contained in the pouch (10) towards and access port 30); and
b) said at least one access member integral with said pouch (10) located at the centre of the bottom portion of said pouch for allowing filling of the pouch (10) with a fluid and access thereto for its delivery, said access member comprising: at least one access port (30) located below the bottom portion of said pouch (10) where said first angle and said second angle meet.

4. A container according to claim 3, wherein said bottom portion terminates in a first angle and a second angle of from 10° to 30° from the centre of said bottom portion and relative to a horizontal plane crossing the centre of said bottom portion.

5. A container according to claim 3, wherein said first angle and second angle are from 10° to 20°.

6. A container according to claims 1-5, wherein said top portion at the periphery of the pouch (10) comprises at least one hole (17) for suspending the pouch (10) when it is in use for delivering the content (22) thereof to the delivery site.

7. A container according to claims 1-6, wherein said bottom portion at the periphery of the pouch (10) comprises one or more holes (21) to facilitate suspending the pouch (10) during the filling process.

8. A container according to claim 1, wherein said first (66) and second (68) flexible polymeric sheets have a generally hexagonal or octagonal configuration to form said pouch (40).

9. A container according to claim 8, wherein
a) said first (66) and second (68) polymeric sheets having a generally hexagonal or octagonal configuration superimposed and sealed together at their periphery (42, 44, 46, 48, 50) to form a pouch (40) defining an interior reservoir, said pouch (40) having a top portion at an angle of the hexagonal or octagonal configuration and a bottom portion at another angle of the hexagonal or octagonal configuration, said top and bottom angles being on opposite sides of each other at the longest distance within the confines of the hexagonal or octagonal configuration, wherein said first and second sheets are non-coplanar to each other; and
b) said access member integral with said pouch (40) located at the centre of the bottom portion of said pouch allowing filling of the pouch (40) with a fluid and access thereto for its delivery, said access member comprising an access port (60) located in said access member.

10. A container according to claims 8-9, wherein said top portion at the periphery of the pouch (40) comprises at least one hole (47) for suspending the pouch (40) when it is in use for delivering the content (22) thereof to the delivery site.

11. A container according to claims 8-10, wherein said bottom portion at the periphery of the pouch (40) comprises one or more holes (51, 53) to facilitate suspending the pouch (40) during the filling process

12. The container according to claim 1, wherein said first (96) and second (98) polymeric sheets have a generally parabolic side configuration to form said pouch (70).

13. The container according to claim 12, wherein said container comprises:
a) first (96) and second (98) collapsibly thin polymeric sheets having a generally parabolic side configuration superimposed and sealed together at their periphery (72, 74, 76) to form
1) an interior reservoir of a concavo-convex shape;
2) a non-collapsible top portion;
3) a non-collapsible bottom portion, and
4) parabolic side portions and
b) an access member.

14. The container according to claim 13, wherein
said top portion comprises:
a centre area where the polymeric sheets sealed together form a hole (77) for suspending the bag during delivery of its content;
two symmetrically positioned rectangular areas extending outwardly from the centre of the bag and sealed at their periphery to render said top portion less flexible than the polymeric sheets which form the interior reservoir; said bottom portion comprises:
two symmetrically positioned rectangular areas extending outwardly from the centre of the bag and sealed at their periphery (72, 74, 76) to render said bottom portion less flexible than the polymeric sheets which form the interior reservoir, said interior reservoir at its bottom portion terminates in a first angle and a second angle of from about 5° to about 45° each from the centre thereof and relative to a horizontal plane crossing the centre of said bottom portion, and comprising one or more holes (81) to facilitate suspending the pouch (70) during the filling process
said parabolic side portions are designed to flex inwardly when the medical bag is filled with a parenteral solution;
said access member, located in the centre of the bottom portion where said first angle and said second angle meet, comprising an access port (90).

15. The container of claim 14, wherein said first angle and second angle are of form 10° to 30°.

16. The container of claim 14, wherein said first angle and second angle are of from 10° to 20°.

17. The container according to claim 1, wherein said first (126) and second (128) polymeric sheets have a generally oval or elliptical configuration to form said pouch (100).

18. The container according to claim 17, wherein
a) said first (126) and second (128) polymeric sheets having a generally oval or elliptical configuration superimposed and sealed together at their periphery (102, 104, 106, 108, 110) to form a pouch (100) defining an interior reservoir forming a concavo-convex shape, said pouch having a top portion and a bottom portion, wherein said first and second sheets are non-coplanar to each other; and
b) an access member integral with said pouch (100) located at the centre of the bottom portion of said pouch (100) allowing filling of the pouch with a fluid and access thereto for its delivery, said access member comprising an access port (120) located in said access member.

19. A container according to claims 17-18, wherein said top portion at the periphery of the pouch (100) comprises at least one hole (107) for suspending the pouch (100) when it is in use for delivering the content (22) thereof to the delivery site.

20. A container according to claims 17-19, wherein said bottom portion at the periphery of the pouch (100) comprises one or more holes (111, 113) to facilitate suspending the pouch (100) during the filling process.

21. The container according to claim 1, wherein said first (156) and second (158) polymeric sheets have a generally spherical configuration to form said pouch (130).

22. The container according to claim 21, wherein
a) first (156) and second (158) flexible polymeric sheets having a generally spherical configuration superimposed and sealed together at their periphery (132, 134, 136, 138, 140) to form a pouch (130) defining an interior reservoir, said pouch (130) having a top portion and a bottom portion, wherein said first and second sheets are non-coplanar to each other forming a concavo-convex shape; and
b) an access member integral with said pouch (130) located at the centre of the bottom portion of said pouch (130) allowing filling of the pouch with a fluid and access thereto for its delivery, said access member comprising an access port (150) located in said access member.

23. A container according to claims 21-22, wherein said top portion at the periphery of the pouch (130) comprises at least one hole (137) for suspending the pouch (130) when it is in use for delivering the content (22) thereof to the delivery site.

24. A container according to claims 21-23, wherein said bottom portion at the periphery of the pouch (130) comprises one or more holes (141, 143) to facilitate suspending the pouch (130) during the filling process

25. The container according to claims 1-24, wherein said first and said second polymeric sheets are made of polyvinyl chloride.

26. The container according to claims 1-24, wherein said first and said second polymeric sheets are made of a polyethylene layer sandwiched between polyester outer layers sealed together by a propylene copolymer.

27. The container according to claims 1-24, wherein said first and said second polymeric sheets are made of polycyclohexanedimethylcyclohexane dicarboxylate.

28. The container according to claims 1-24, wherein said first and said second polymeric sheets are made of ethyl vinyl acetate.

29. The container according to claims 1-24, wherein said first and said second polymeric sheets are made of a transparent, UV rays absorbent material selected from the group consisting of: copolyester elastomers, ethylene methocrylate, ethylene vinyl acetate, ethylene vinyl alcohol, low density polyethylene, nylon/polypropylene, polyester, polyolefin, polypropylene, polyethylene and polyvinylchloride containing a UV blocking or UV absorbing agent.
